# EUROPEAN PATENT SPECIFICATION

(11) **EP 3 962 438 B1**
(45) Date of publication and mention of the grant of the patent: **06.09.2023**
(21) Application number: 20723111.9
(22) Date of filing: 29.04.2020
(51) Int. Cl.: A61K 8/19, A61K 8/02, A61Q 11/00

(54) **MAGNESIUM ION-CONTAINING MATERIALS AS WHITE PIGMENTS IN ORAL CARE COMPOSITIONS**
MAGNESIUMIONENHALTIGE MATERIALIEN ALS WEISSPIGMENTE IN MUNDPFLEGEZUSAMMENSETZUNGEN
MATÉRIAUX CONTENANT DES IONS DE MAGNÉSIUM EN TANT QUE PIGMENTS BLANCS DANS DES COMPOSITIONS DE SOINS BUCCAUX

(30) Priority: 03.05.2019 EP 19172540
(43) Date of publication of application: 09.03.2022
(73) Proprietor: Omya International AG, 4665 Oftringen (CH)
(72) Inventor: KELLER, Tobias, 5722 Gränichen (CH); BUDDE, Tanja, 4805 Brittnau (CH); RENTSCH, Samuel, 3095 Spiegel bei Bern (CH)
(74) Representative: Maiwald GmbH
(86) International application number: PCT/EP2020/061952
(87) International publication number: WO 2020/225064

(56) References cited:
- WO-A1-00/28977
- WO-A1-2014/148997
- US-A- 4 405 599
- US-A- 5 645 821
- US-B1- 6 669 928

## Description

The present invention relates to an oral care composition comprising a magnesium ion-containing material in an amount from 0.1 to 40 wt.-%, based on the total weight of the composition as well as the use of a magnesium ion-containing material as opacifying agent and/or whitening pigment in oral care compositions.

A wide variety of oral care products is used to clean, protect and groom the tooth and to maintain its structure. For example, WO 2000/010520 A1 refers to a toothpaste comprising, in a liquid or pasty medium, particulate calcium carbonate as the main abrasive cleaning agent, characterized in that the particulate calcium carbonate comprises a mixture of 75-92.5 % by weight of the mixture fine of particulate calcium carbonate with a weight average particle size of between 1 and 15 microns, and 7.5-25 % by weight of the mixture of coarse particulate calcium carbonate with a weight average particle size of between 30 and 120 microns. EP 2 461 794 A2 refers to a toothpaste composition comprising a binder, an abrasive, a foaming agent, water, and polyethylene glycol, wherein said binder comprises semirefined iota carrageenan. US 2009/0117058 A1 refers to a whitening toothpaste composition with improved preservativeness and a sustained tooth whitening effect, characterized by containing peroxide and purified silica. WO 2014/059678 A1 refers to a toothpaste composition comprising: an orally acceptable vehicle, an abrasive comprising calcium carbonate; and a binder system comprising guar gum and at least one cellulose polymer; wherein the binder system is substantially free of magnesium aluminum silicate. US 4,254,101 A refers to a toothpaste composition comprising: (A) from about 6% to 45% of a silica dental abrasive; (B) from about 30% to 70% of a humectant; (C) from about 0.03% to 1.0% of a carboxyvinyl polymer; and (D) from about 10% to 45% of water; said composition providing a pH of from about 4.0 to 8.0 when slurried with water in a 3:1 water/composition weight ratio. WO 2013/007571 A2 refers to a toothpaste composition comprising: (i) a calcium based abrasive; (ii) a copolymer of vinylmethyl ether and maleic acid; and, (iii) a clay wherein ratio of said Calcium based abrasive to said copolymer of vinyl methyl ether and maleic anhydride is at least 1 : 0.0075 and ratio of said calcium based abrasive to said clay is at least 1 : 0.02. WO 2012/143220 A1 describes a composition that is suitable for remineralisation and whitening of teeth, which comprises a calcium source and regeneration-source calcium salt. A dentifrice composition comprising a water insoluble and/or slightly water-soluble calcium source and an organic acid, or its physiologically acceptable salt, is described in WO 2013/034421 A2. WO 2012/031786 A2 relates to oral care compositions with composite particle actives having a core and a coating, whereby the coating interacts with phosphate ions to produce calcium and phosphate reaction products that are suitable to adhere to tooth enamel and/or dentine to improve the characteristics of teeth. US 5 645 821 A discloses an oral hygiene composition with magnesium carbonate and magnesium hydroxide as fine powders. Basic pH and mild abrasive properties characterise the product.

Usually such products are modified in their appearance in order to satisfy consumer expectations. For example, from a consumer perspective there is a demand for white and opaque products. Currently, titanium dioxide is broadly applied as white pigment in oral care products. For example, US 3,935,304 A refers to a toothpaste containing at least about 25% by weight dispersed particles of sodium bicarbonate and a polishing agent system comprising titanium dioxide powder having a particle size less than about two microns, the amount of titanium dioxide particles being more than about 0.1% of the weight of the toothpaste, said particles dispersed in a vehicle containing sufficient liquids, said vehicle consisting essentially of about 5 to 35% water and sufficient viscous water miscible polyol humectant or mixtures thereof, and a sufficient amount of gelling or thickening agent to impart to the toothpaste the pasty consistency, body and the non-tacky nature which is characteristic of conventional dental creams or toothpastes, said sodium bicarbonate being primarily in an undissolved solid state, said dental cream having a granular textured appearance comprising a substantially dispersed non-crystalline appearing granulate of macroscopic crystalline bicarbonate granules in an otherwise smooth continuous matrix.

However, there are strong concerns regarding the use of titanium dioxide in such compositions due to the possible health risks of titanium dioxide and especially of such nanoparticles in said products. Calcium carbonates are also known as white pigments in a wide variety of products. Calcium carbonates such as ground calcium carbonate, precipitated calcium carbonate, and mixtures thereof have a major disadvantage compared to titanium dioxide and thus are not considered as material of choice in oral care products. In particular, oral care products are typically provided with fluoride ions for preventing tooth decay and caries. This fluoride may be provided as sodium fluoride. However, fluoride ions strongly absorb on the calcium carbonate surface as calcium fluoride and thus making it unavailable for the interaction with the teeth.

However, the provision of an oral care composition being free of titanium dioxide remains of interest to the skilled man. Furthermore, it is desired to provide an oral care composition providing a sufficient whiteness and/or opacity. Furthermore, it is desired to provide an oral care composition providing a high availability of fluoride ions in the composition.

Accordingly, it is an object of the present invention to provide an oral care composition, preferably being free of titanium dioxide. A further object of the present invention is to provide an oral care composition providing a sufficient whiteness and/or opacity. A further object of the present invention is to provide an oral care composition providing a high availability of fluoride ions in the composition, especially compared to compositions comprising calcium carbonate.

The foregoing objects and other objects are solved by the subject-matter as defined herein in the independent claims.

According to one aspect of the present invention, an oral care composition comprising a magnesium ion-containing material in an amount from 0.1 to 40 wt.-%, based on the total weight of the composition, is provided.

According to another aspect of the present invention, the use of a magnesium ion-containing material as opacifying agent and/or whitening pigment in oral care compositions is provided.

The inventors surprisingly found out that the foregoing oral care composition provides a sufficient whiteness and/or opacity as well as a high availability of fluoride ions, while it is free of titanium dioxide. More precisely, the inventors found out that the whiteness and/or opacity of an oral care composition is sufficient and further provides a high availability of fluoride ions if a magnesium ion-containing material in an amount from 0.1 to 40 wt.-% is used in the composition.

Advantageous embodiments of the inventive oral care composition and the use are defined in the corresponding sub-claims.

According to one embodiment, the magnesium ion-containing material is selected from the group consisting of anhydrous magnesium carbonate or magnesite (MgCO₃), hydromagnesite (Mg₅(CO₃)₄(OH)₂ · 4H₂O), artinite (Mg₂(CO₃)(OH)₂ · 3H₂O), dypingite (Mg₅(CO₃)₄(OH)₂ . 5H₂O), giorgiosite (Mg₅(CO₃)₄(OH)₂ · 5H₂O), pokrovskite (Mg₂(CO₃)(OH)₂ · 0.5H₂O), barringtonite (MgCO₃ · 2H₂O), lansfordite (MgCO₃ · 5H₂O), nesquehonite (MgCO₃ · 3H₂O), brucite (Mg(OH)₂), dolomite (CaMg(CO₃)₂), dolocarbonate and mixtures thereof, preferably selected from anhydrous magnesium carbonate or magnesite (MgCO₃), dolomite (CaMg(CO₃)₂), hydromagnesite (Mg₅(CO₃)₄(OH)₂ · 4H₂O), brucite (Mg(OH)₂) and mixtures thereof.

According to another embodiment, the magnesium ion-containing material is in form of particles having a) a volume median grain diameter (*d*₅₀) of ≥ 150 nm, preferably from 150 nm to 20 µm, more preferably from 0.2 to 15 µm, even more preferably from 0.5 to 10 µm, and most preferably from 1 to 5 µm, as determined by laser diffraction, and/or b) a volume determined top cut particle size (*d*₉₈) of equal to or less than 30 µm, preferably from 2to 30 µm, more preferably from 5 to 20, and most preferably from 8 to 18 µm, as determined by laser diffraction.

According to yet another embodiment, the magnesium ion-containing material has a whiteness determined as CIELAB L^{∗} of ≥ 90 %, preferably > 95 %, more preferably > 98 % and most preferably > 98.5 % and measured dry according to EN ISO 11664-4:2010.

According to one embodiment, the magnesium ion-containing material is in form of particles having a BET specific surface area in the range from 2 to 200 m²/g, preferably from 10 to 100 m²/g, and most preferably from 12 to 75 m²/g, measured using nitrogen and the BET method according to ISO 9277:2010.

According to yet another embodiment, the oral care composition further comprises a fluoride compound, preferably the fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride.

According to one embodiment, the oral care composition further comprises a remineralisation and/or whitening agent, preferably selected from the group consisting of silica, hydroxylapatite, e.g. nano-hydroxylapatite, calcium carbonate, e.g. amorphous calcium carbonate, ground calcium carbonate, precipitated calcium carbonate, surface-reacted calcium carbonate and combinations thereof, calcium silicate and mixtures thereof.

According to another embodiment, the oral care composition is a toothpaste, a toothgel, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash, preferably a chewable gum, a lozenge, a toothpaste, a toothpowder, or a mouthwash, and most preferably a toothpaste.

According to yet another embodiment, the oral care composition has a pH between 6.8 and 10, preferably between 7.5 and 9 and most preferably between 8 and 9.

According to one embodiment, the oral care composition comprises the magnesium ion-containing material in an amount from 0.5 to 10 wt.-%, based on the total weight of the composition.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

In the following, preferred embodiments of the inventive oral care composition will be set out in more detail. It is to be understood that these embodiments and details also apply to the inventive use as far as applicable.

### Oral care composition

According to the present invention, an oral care composition is provided. The oral care composition comprises a magnesium ion-containing material in an amount from 0.1 to 40 wt.-%, based on the total weight of the composition.

It is appreciated that the term "magnesium ion-containing material" refers to a material that comprises at least 38 wt.-% of a magnesium compound. In one embodiment, the magnesium ion-containing material comprises at least 38 wt.-%, preferably between 38 and 100 wt.-%, more preferably between 38 and 99.95 wt.-%, e.g. from 38 to 55 wt.-%, based on the total dry weight of the material, of the magnesium compound. In another embodiment, the magnesium ion-containing material comprises, at least 85 wt.-%, preferably between 85 and 100 wt.-%, more preferably between 90 and 99.95 wt.-%, based on the total dry weight of the material, of the magnesium compound. Thus, it is to be noted that the magnesium ion-containing material may further comprise impurities typically associated with the type of material used. For example, the magnesium ion-containing material may further comprise impurities such as calcium ion-containing materials like calcium hydroxide, calcium carbonate and mixtures thereof.

For example, if the magnesium ion-containing material comprises the magnesium compound in an amount of at least 38 wt.-%, preferably between 38 and 100 wt.-%, more preferably between 38 and 99.95 wt.-%, e.g. from 38 to 45 wt.-%, based on the total dry weight of the material, the impurities such as calcium ion-containing materials like calcium hydroxide, calcium carbonate and mixtures thereof are present in amounts of less than 62 wt.-%, preferably between 0 and 62 wt.-%, more preferably between 0.05 and 62 wt.-%, e.g. from 45 to 62 wt.-%, based on the total dry weight of the material. If the magnesium ion-containing material comprises the magnesium compound in an amount of at least 85 wt.-%, preferably between 85 and 100 wt.-%, more preferably between 90 and 99.95 wt.-%, based on the total dry weight of the material, the impurities such as calcium ion-containing materials like calcium hydroxide, calcium carbonate and mixtures thereof are present in amounts of less than 15 wt.-% and most preferably from 0.05 to 10 wt.-%, based on the total dry weight of the material. It is further appreciated that the magnesium ion-containing material may be a mineral phase comprising calcium and magnesium ions, such as dolomite (MgCa(CO₃)₂).

The magnesium ion-containing material can be a naturally occurring or synthetic magnesium ion-containing material.

According to one embodiment of the present invention the naturally occurring magnesium ion-containing material may be obtained by dry grinding. According to another embodiment of the present invention, the naturally occurring magnesium ion-containing material may be obtained by wet grinding and optionally subsequent drying.

In general, the grinding step can be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the magnesium ion-containing material is obtained by wet-grinding, the grinding step may be performed under conditions such that autogenous grinding takes place and/or by horizontal ball milling, and/or other such processes known to the skilled man. The wet processed ground magnesium ion-containing material thus obtained may be washed and dewatered by well-known processes, e.g. by flocculation, filtration or forced evaporation prior to drying. The subsequent step of drying may be carried out in a single step such as spray drying, or in at least two steps. It is also common that such a mineral material undergoes a beneficiation step (such as a flotation, bleaching or magnetic separation step) to remove impurities.

Synthetic magnesium ion-containing materials in the meaning of the present invention can be obtained by processes well known in the art. For instance, US 1,361,324, US 935,418, GB 548,197 and GB 544,907 generally describe the formation of aqueous solutions of magnesium bicarbonate (typically described as "Mg(HCO₃)₂"), which is then transformed by the action of a base, e.g., magnesium hydroxide, to form hydromagnesite. Other processes described in the art suggest to prepare compositions containing both, hydromagnesite and magnesium hydroxide, wherein magnesium hydroxide is mixed with water to form a suspension which is further contacted with carbon dioxide and an aqueous basic solution to form the corresponding mixture; cf. for example US 5,979,461. EP 0 526 121 describes a calcium-magnesium carbonate composite consisting of calcium carbonate and magnesium carbonate hydroxide and a method for the preparation thereof. Furthermore, GB 594,262 relates to a method and apparatus for treating magnesia-containing materials, such as magnesium and calcium carbonate materials for obtaining respective carbonates in discrete and separate forms, by controlled carbonation such that the magnesium and calcium carbonates may be separated by mechanical means and with attainment of special utilities in separated products. US 2007194276 describes a method of reductively bleaching a mineral slurry comprising adding in the mineral slurry an effective amount of a formamidine sulfinic acid (FAS) and an effective amount of a borohydride to reductively bleach the mineral slurry.

For example, the magnesium ion-containing material encompasses a naturally occurring or synthetic magnesium ion-containing material selected from the group consisting of anhydrous magnesium carbonate or magnesite (MgCO₃), hydromagnesite (Mg₅(CO₃)₄(OH)₂ · 4H₂O), artinite (Mg₂(CO₃)(OH)₂ . 3H₂O), dypingite (Mg₅(CO₃)₄(OH)₂ . 5H₂O), giorgiosite (Mg₅(CO₃)₄(OH)₂ . 5H₂O), pokrovskite (Mg₂(CO₃)(OH)₂ · 0.5H₂O), barringtonite (MgCO₃ · 2H₂O), lansfordite (MgCO₃ · 5H₂O), nesquehonite (MgCO₃ · 3H₂O), brucite (Mg(OH)₂), dolomite (CaMg(CO₃)₂), dolocarbonate and mixtures thereof, preferably selected from anhydrous magnesium carbonate or magnesite (MgCO₃), dolomite (CaMg(CO₃)₂), hydromagnesite (Mg₅(CO₃)₄(OH)₂ · 4H₂O), brucite (Mg(OH)₂) and mixtures thereof.

In the meaning of the present invention, the term "dolocarbonate" refers to a composite material comprising a magnesium mineral, preferably hydromagnesite (Mg₅(CO₃)₄(OH)₂ · 4H₂O), and calcium carbonate agglomerated at primary particle level. Such dolocarbonates are for examples described in WO 2013/139957 A1 and WO 2015/039994 A1, which are thus incorporated by references.

Preferably, the magnesium ion-containing material encompasses a naturally occurring or synthetic magnesium ion-containing material selected from the group consisting of anhydrous magnesium carbonate or magnesite (MgCO₃), hydromagnesite (Mg₅(CO₃)₄(OH)₂ · 4H₂O), nesquehonite (MgCO₃ · 3H₂O), brucite (Mg(OH)₂), dolomite (CaMg(CO₃)₂), dolocarbonate and mixtures thereof. For example, the magnesium ion-containing material comprises the naturally occurring or synthetic magnesium carbonate selected from the group consisting of anhydrous magnesium carbonate or magnesite (MgCO₃), hydromagnesite (Mg₅(CO₃)₄(OH)₂ · 4H₂O), nesquehonite (MgCO₃ · 3H₂O), brucite (Mg(OH)₂), dolomite (CaMg(CO₃)₂), dolocarbonate and mixtures thereof in an amount of at least 80 wt.-%, more preferably at least 85 wt.-%, even more preferably between 85 and 100 wt.-%, and most preferably between 90 and 99.95 wt.-%, based on the total dry weight of the material.

In one embodiment, the magnesium ion-containing material comprises anhydrous magnesium carbonate or magnesite (MgCO₃) and/or dolomite (CaMg(CO₃)₂) and/or, hydromagnesite (Mg₅(CO₃)₄(OH)₂ · 4H₂O) and/or brucite (Mg(OH)₂), preferably synthetic hydromagnesite (Mg₅(CO₃)₄(OH)₂ · 4H₂O) and/or brucite (Mg(OH)₂) and/or naturally occurring anhydrous magnesium carbonate or magnesite (MgCO₃) and/or dolomite (CaMg(CO₃)₂). Preferably, the magnesium ion-containing material comprises anhydrous magnesium carbonate or magnesite (MgCO₃) and/or dolomite (CaMg(CO₃)₂) and/or hydromagnesite (Mg₅(CO₃)₄(OH)₂ . 4H₂O) and/or brucite (Mg(OH)₂), preferably synthetic hydromagnesite (Mg₅(CO₃)₄(OH)₂ · 4H₂O) and/or brucite (Mg(OH)₂) and/or naturally occurring anhydrous magnesium carbonate or magnesite (MgCO₃) and/or dolomite (CaMg(CO₃)₂), in an amount of at least 80 wt.-%, more preferably at least 85 wt.-%, even more preferably between 85 and 100 wt.-%, and most preferably between 90 and 99.95 wt.-%, based on the total dry weight of the material.

For example, the magnesium ion-containing material comprises anhydrous magnesium carbonate or magnesite (MgCO₃) or dolomite (CaMg(CO₃)₂) or hydromagnesite (Mg₅(CO₃)₄(OH)₂ · 4H₂O) or brucite (Mg(OH)₂), e.g. synthetic hydromagnesite (Mg₅(CO₃)₄(OH)₂ · 4H₂O) or brucite (Mg(OH)₂), preferably hydromagnesite (Mg₅(CO₃)₄(OH)₂ · 4H₂O), e.g. synthetic hydromagnesite (Mg₅(CO₃)₄(OH)₂ · 4H₂O) or naturally occurring anhydrous magnesium carbonate or magnesite (MgCO₃) or dolomite (CaMg(CO₃)₂). For example, the magnesium ion-containing material comprises anhydrous magnesium carbonate or magnesite (MgCO₃), e.g. naturally occurring anhydrous magnesium carbonate or magnesite (MgCO₃). Alternatively, the magnesium ion-containing material comprises dolomite (CaMg(CO₃)₂), e.g. naturally occurring dolomite (CaMg(CO₃)₂). In one embodiment, the magnesium ion-containing material comprises anhydrous magnesium carbonate or magnesite (MgCO₃) or dolomite (CaMg(CO₃)₂) or hydromagnesite (Mg₅(CO₃)₄(OH)₂ . 4H₂O) or brucite (Mg(OH)₂), e.g. synthetic hydromagnesite (Mg₅(CO₃)₄(OH)₂ . 4H₂O) or brucite (Mg(OH)₂) or naturally occurring anhydrous magnesium carbonate or magnesite (MgCO₃) or dolomite (CaMg(CO₃)₂), , in an amount of at least 80 wt.-%, more preferably at least 85 wt.-%, even more preferably between 85 and 100 wt.-%, and most preferably between 90 and 99.95 wt.-%, based on the total dry weight of the material.

In one embodiment, the magnesium ion-containing material consists of anhydrous magnesium carbonate or magnesite (MgCO₃) and/or dolomite (CaMg(CO₃)₂), e.g. naturally occurring anhydrous magnesium carbonate or magnesite (MgCO₃) or dolomite (CaMg(CO₃)₂).

In an alternative embodiment, the magnesium ion-containing material consists of hydromagnesite (Mg₅(CO₃)₄(OH)₂ · 4H₂O) or brucite (Mg(OH)₂), e.g. synthetic hydromagnesite (Mg₅(CO₃)₄(OH)₂ · 4H₂O) or brucite (Mg(OH)₂), preferably hydromagnesite (Mg₅(CO₃)₄(OH)₂ · 4H₂O), e.g. synthetic hydromagnesite (Mg₅(CO₃)₄(OH)₂ · 4H₂O).

It is appreciated that the oral care composition is preferably free of nanosized (white) pigment particles such as nanosized titanium dioxide. It is thus preferred that the magnesium ion-containing material does not comprise particles having a primary particle size of < 100 nm.

According to one embodiment of the present invention, the magnesium ion-containing material is in form of particles having a volume median grain diameter (*d*₅₀) of ≥ 150 nm, preferably from 150 nm to 20 µm, more preferably from 0.2 to 15 µm, even more preferably from 0.5 to 10 µm, and most preferably from 1 to 5 µm, as determined by laser diffraction. According to a further embodiment of the present invention, the magnesium ion-containing material is in form of particles having a volume determined top cut particle size (*d*₉₈) of equal to or less than 30 µm, preferably from 2 to 30 µm, more preferably from 5 to 20, and most preferably from 8 to 18 µm, as determined by laser diffraction.

Thus, the magnesium ion-containing material is in form of particles preferably having
a) a volume median grain diameter (*d*₅₀) of ≥ 150 nm, preferably from 150 nm to 20 µm, more preferably from 0.2 to 15 µm, even more preferably from 0.5 to 10 µm, and most preferably from 1 to 5 µm, as determined by laser diffraction, and
b) a volume determined top cut particle size (*d*₉₈) of equal to or less than 30 µm, preferably from 2 to 30 µm, more preferably from 5 to 20, and most preferably from 8 to 18 µm, as determined by laser diffraction.

In one embodiment, the magnesium ion-containing material is in form of particles having a volume median grain diameter (*d*₅₀) in the range from 1 to 5 µm, as determined by laser diffraction, and a volume determined top cut particle size (*d*₉₈) in the range from 8 to 18 µm, as determined by laser diffraction.

For example, the magnesium ion-containing material comprises anhydrous magnesium carbonate or magnesite (MgCO₃) or dolomite (CaMg(CO₃)₂) or hydromagnesite (Mg₅(CO₃)₄(OH)₂ . 4H₂O) or brucite (Mg(OH)₂), e.g. synthetic hydromagnesite (Mg₅(CO₃)₄(OH)₂ . 4H₂O) or brucite (Mg(OH)₂) or naturally occurring anhydrous magnesium carbonate or magnesite (MgCO₃) or dolomite (CaMg(CO₃)₂), and has a volume median grain diameter (*d*₅₀) in the range from 1 to 5 µm, as determined by laser diffraction, and a volume determined top cut particle size (*d*₉₈) in the range from 8 to 18 µm, as determined by laser diffraction.

In one embodiment, the magnesium ion-containing material comprises anhydrous magnesium carbonate or magnesite (MgCO₃) or dolomite (CaMg(CO₃)₂) or hydromagnesite (Mg₅(CO₃)₄(OH)₂ · 4H₂O) or brucite (Mg(OH)₂), e.g. synthetic hydromagnesite (Mg₅(CO₃)₄(OH)₂ · 4H₂O) or brucite (Mg(OH)₂) or naturally occurring anhydrous magnesium carbonate or magnesite (MgCO₃) or dolomite (CaMg(CO₃)₂), in an amount of at least 80 wt.-%, more preferably at least 85 wt.-%, even more preferably between 85 and 100 wt.-%, and most preferably between 90 and 99.95 wt.-%, based on the total dry weight of the material and has a volume median grain diameter (*d*₅₀) in the range from 1 to 5 µm, as determined by laser diffraction, and a volume determined top cut particle size (*d*₉₈) in the range from 8 to 18 µm, as determined by laser diffraction.

Volume determined median grain diameter *d*₅₀ (or *d*₅₀(vol)) and the volume determined top cut particle size *d*₉₈ (or *d*₉₈(vol)) was evaluated using a Malvern Mastersizer 3000 Laser Diffraction System (Malvern Instruments Plc., Great Britain) equipped with a Hydro LV system. The *d*₅₀(vol) or *d*₉₈(vol) value indicates a diameter value such that 50 % or 98 % by volume, respectively, of the particles have a diameter of less than this value. The powders were suspended in 0.1 wt.-% Na₄O₇P₂ solution. 10 mL of 0.1 wt.-% Na₄O₇P₂ was added to the Hydro LV tank, then the sample slurry was introduced until an obscuration between 10-20 % was achieved. Measurements were conducted with red and blue light for 10 s each. For the analysis of the raw data, the models for non-spherical particle sizes using Mie theory was utilized, and a particle refractive index of 1.57, a density of 2.70 g/cm³, and an absorption index of 0.005 was assumed. The methods and instruments are known to the skilled person and are commonly used to determine particle size distributions of fillers and pigments.

Additionally or alternatively, the magnesium ion-containing material has a whiteness determined as CIELAB L* of ≥ 90 %, preferably > 95 %, more preferably > 98 % and most preferably > 98.5 % and measured dry according to EN ISO 11664-4:2010.

In one embodiment, the magnesium ion-containing material is in form of particles having a BET specific surface area in the range from 2 to 200 m²/g, preferably from 10 to 100 m²/g, and most preferably from 12 to 75 m²/g, measured using nitrogen and the BET method according to ISO 9277:2010.

The "specific surface area" (expressed in m²/g) of a material as used throughout the present application can be determined by the Brunauer Emmett Teller (BET) method with nitrogen as adsorbing gas and by use of a ASAP 2460 instrument from Micromeritics. The method is well known to the skilled person and defined in ISO 9277:2010. Samples are conditioned at 150 °C under vacuum for a period of 60 min prior to measurement.

In one embodiment, the magnesium ion-containing material contains up to 25 000 ppm Ca²⁺ ions. For example, the magnesium ion-containing material contains up to 20 000 ppm, more preferably up to 15 000 ppm and most preferably up to 5 000 ppm Ca²⁺ ions.

It is preferred that the magnesium ion-containing material according to the present invention has not been obtained by treating the surface of the magnesium ion-containing material with a surface treatment agent and thus is not surface-treated.

According to the present invention, the oral care composition comprises the magnesium ion-containing material in an amount from 0.1 to 40 wt.-%, based on the total weight of the composition.

According to one embodiment of the present invention, the magnesium ion-containing material is present in an amount from 0.1 to 30 wt.-%, preferably from 0.1 to 20 wt.-%, more preferably from 0.5 to 15 wt.-%, and most preferably from 0.5 to 10 wt.-%, based on the total weight of the composition.

According to another embodiment, the oral care composition may comprise at least one whitening agent and/or remineralization agent. It is appreciated that such whitening agent is typically not added in order to whiten the oral care composition (as the magnesium ion-containing material) but rather to whiten the teeth.

The whitening agent can be a bleaching agent, an abrasive, or a remineralisation agent, and is preferably selected from the group consisting of hydrogen peroxide, carbamide peroxide, hydroxylapatite, calcium carbonate, and mixtures thereof.

According to one embodiment of the present invention, the at least one remineralisation and/or whitening agent is selected from the group consisting of silica, hydroxylapatite, e.g. nano-hydroxylapatite, calcium carbonate, e.g. amorphous calcium carbonate, ground calcium carbonate, precipitated calcium carbonate, surface-reacted calcium carbonate and combinations thereof, calcium silicate and mixtures thereof.

According to one embodiment, the remineralisation and/or whitening agent preferably has a weight median particle size *d*₅₀ from 10 nm to 100 µm, preferably from 0.1 to 50 µm, more preferably from 1 to 20 µm, and most preferably from 2 to 10 µm.

The at least one remineralisation and/or whitening agent, if present, can be present in the oral care composition in an amount from 1 to 20 wt.-%, preferably from 1.5 to 15 wt.-%, more preferably from 2 to 10 wt.-%, based on the total weight of the composition.

According to one embodiment, the oral care composition of the present invention comprises from 0.1 to 40 wt.-% of the magnesium ion-containing material and from 1 to 20 wt.-% of a remineralisation and/or whitening agent, based on the total weight of the composition.

The oral care composition of the present invention can be, for example, a toothpaste, a toothgel, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash.

According to one embodiment of the present invention, the oral care composition is a chewable gum, a lozenge, a toothpaste, a toothpowder, or a mouthwash, and preferably a toothpaste.

According to another preferred embodiment, the oral care composition is a toothpaste, a toothpowder, or a mouthwash and the magnesium ion-containing material is in form of particles having a volume median grain diameter (*d*₅₀) of ≥ 150 nm, preferably from 150 nm to 20 µm, more preferably from 0.2 to 15 µm, even more preferably from 0.5 to 10 µm, and most preferably from 1 to 5 µm, as determined by laser diffraction, and a volume determined top cut particle size (*d*₉₈) of equal to or less than 30 µm, preferably from 2 to 30 µm, more preferably from 5 to 20, and most preferably from 8 to 18 µm, as determined by laser diffraction.

Preferably, the oral care composition is a toothpaste, a toothpowder, or a mouthwash and the magnesium ion-containing material is in form of particles having a volume median grain diameter (*d*₅₀) of from 1 to 5 µm, as determined by laser diffraction, and a volume determined top cut particle size (*d*₉₈) of from 8 to 18 µm, as determined by laser diffraction.

According to one embodiment of the present invention, the oral care composition has a pH between 6.8 and 10, preferably between 7.5 and 9 and most preferably between 8 and 9.

The inventive oral care composition can be used in combination with a fluoride compound. The inventors surprisingly found that due to the presence of the magnesium ion-containing material in the inventive oral care composition a high availability of fluoride ions in the composition is provided. Accordingly, the actual amount of fluoride compound(s) in the composition can be decreased, especially compared to compositions comprising calcium carbonate.

According to a preferred embodiment, the oral care composition further comprises a fluoride compound. The fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof. Preferably, the fluoride compound is sodium monofluorophosphate and/or sodium fluoride.

Good results can be achieved by employing an amount of fluoride compound to provide available fluoride ion in the range of 300 to 2 000 ppm in the oral care composition, preferably about 1 450 ppm.

In addition to the magnesium ion-containing material, the optional remineralisation and/or whitening agent, and the optional fluoride compound, the oral care composition may further comprise additives typically used in the composition to be prepared, such as bioadhesive polymers, surfactants, binders, humectants, desensitising agents, flavouring agents, sweetening agents and/or water. Such compounds are well known in the art.

According to one embodiment of the present invention, the oral care composition comprises a bioadhesive polymer. The bioadhesive polymer may include any polymer that promotes adhesion of any of the components of the oral care composition to teeth or tooth surface and remains on the teeth or tooth surface for an extended period of time, for example, 1 hour, 3 hours, 5 hours, 10 hours or 24 hours. In certain embodiments, the bioadhesive polymer may become more adhesive when the oral care composition is moistened with, for example, water or saliva. In other embodiments, the bioadhesive polymer is a material or combination of materials that enhance the retention of the active ingredient on the teeth or a tooth surface onto which the composition is applied. Such bioadhesive polymers include, for example, hydrophilic organic polymers, hydrophobic organic polymers, silicone gums, silicas, and combinations thereof. According to one embodiment, the bioadhesive polymer is selected from the group consisting of hydroxyethyl methacrylate, PEG/PPG copolymers, polyvinylmethylether/maleic anhydride copolymers, polyvinylpyrrolidone (PVP), cross-linked PVP, shellac, polyethylene oxide, methacrylates, acrylates copolymers, methacrylic copolymers, vinylpyrrolidone/vinyl acetate copolymers, polyvinyl caprolactum, polylactides, silicone resins, silicone adhesives, chitosan, milk proteins (casein), amelogenin, ester gum, and combinations thereof.

Suitable surfactants are generally anionic organic synthetic surfactants throughout a wide pH range. Representative of such surfactants used in the range of about 0.5 to 5 wt.-%, based on the total weight of the oral care composition, are water-soluble salts of C₁₀-C₁₈ alkyl sulphates, such as sodium lauryl sulphate, of sulphonated monoglycerides of fatty acids, such as sodium monoglyceride sulphonates, of fatty acid amides of taurine, such as sodium N-methyl-N-palmitoyltauride, and of fatty acid esters of isethionic acid, and aliphatic acylamides, such as sodium N-lauroyl sarcosinate. However, surfactants obtained from natural sources such as cocamidopropyl betaine may also be used.

Suitable binders or thickening agents to provide the desired consistency are, for example, hydroxyethylcellulose, sodium carboxymethylcellulose, natural gums, such as gum karaya, gum arabic, gum tragacanth, xanthan gum or cellulose gum. Generally, from 0.5 to 5 wt.-%, based on the total weight of the oral care composition, can be used.

Desensitising agents can be selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof.

Various humectants known to the skilled person can be used, such as glycerine, sorbitol and other polyhydric alcohols, for example, in an amount from 20 to 40 wt.-%, based on the total weight of the oral care composition. Examples of suitable flavouring agents include oil of wintergreen, oil of spearmint, oil of peppermint, oil of clove, oil of sassafras and the like. Saccharin, aspartame, dextrose, or levulose can be used as sweetening agents, for example, in an amount from 0.01 to 1 wt.-%, based on the total weight of the oral care composition. Preservatives such as sodium benzoate may be present in an amount from 0.01 to 1 wt.-%, based on the total weight of the oral care composition. Colorants may also be added to the oral care composition, for example, in an amount from 0.01 to 1.5 wt.-%, based on the total weight of the oral care composition.

According to one embodiment of the present invention, the oral care composition is a toothpaste. The toothpaste may be produced by a method comprising the following steps:
I) providing a mixture of water and humectant(s), and optionally at least one of a thickener, a preservative, a fluoride compound, and a sweetener,
II) adding the magnesium ion-containing material in an amount from 0.1 to 40 wt.-%, based on the total weight of the composition, and optionally a colorant, to the mixture of step I),
III) adding a surfactant to the mixture of step II), and
IV) optionally, adding a flavouring agent to the mixture of step III).

However, a toothpaste of the present invention may also be produced by any other method known to the skilled person.

The oral care composition of the present invention may be used in professional, in-office treatment or in at home treatment.

According to one embodiment, the oral care composition is used in a method comprising the step of administering to at least one tooth of a patient a therapeutically effective amount of the oral care composition at least once a day, preferably twice a day and more preferably three-times a day. A "therapeutically effective" amount of the oral care composition is an amount that is sufficient to have the desired therapeutic or prophylactic effect in the human subject to whom the composition is administered, without undue adverse side effects (such as toxicity, irritation, or allergic response), commensurate with a reasonable benefit/risk ratio when used in the manner of this invention. The specific effective amount will vary with such factors as the particular condition being treated, the physical condition of the subject, the nature of concurrent therapy (if any), the specific dosage form, and the specific oral care composition employed.

According to one embodiment, the oral care composition of the present invention is used in a method comprising the step of applying the composition to at least one tooth of a patient for an effective amount of time, preferably the composition remains on the at least one tooth for at least 1 min, at least 15 min, at least 30 min, at least 1 hour, at least 2 hours, at least 12 hours or at least 24 hours.

According to a preferred embodiment of the present invention, the oral care composition does not contain an oxidative whitening compound.

### The use

It was found that a magnesium ion-containing material according to the present invention can be used as opacifying agent and/or whitening pigment in oral care compositions.

According to one embodiment of the present invention, a magnesium ion-containing material is provided that can be used as opacifying agent in oral care compositions.

According to another embodiment of the present invention, a magnesium ion-containing material is provided that can be used as whitening pigment in oral care compositions.

According to another embodiment of the present invention, a magnesium ion-containing material is provided that can be used as opacifying agent and whitening pigment in oral care compositions.

With regard to the definition of the magnesium ion-containing material, the oral care composition, and preferred embodiments thereof, reference is made to the statements provided above when discussing the technical details of the oral care composition of the present invention.

It is appreciated that the magnesium ion-containing material can be used as whitening pigment and thus is intended to impart whiteness to the oral care composition. i.e. the magnesium ion-containing material does not whiten the teeth.

It was surprisingly found by the inventors that the magnesium ion-containing material also provides a high availability of fluoride ions in an oral care composition, especially compared to oral care compositions comprising calcium carbonate.

The scope and interest of the present invention will be better understood based on the following examples which are intended to illustrate certain embodiments of the present invention.

### Examples

### 1. Measurement methods

In the following, measurement methods implemented in the examples are described.

### Particle size distribution

Volume determined median particle size *d*₅₀(vol) and the volume determined top cut particle size *d*₉₈(vol) was evaluated using a Malvern Mastersizer 3000 Laser Diffraction System (Malvern Instruments Plc., Great Britain) equipped with a Hydro LV system. The *d*₅₀(vol) or *d*₉₈(vol) value indicates a diameter value such that 50 % or 98 % by volume, respectively, of the particles have a diameter of less than this value. The powders were suspended in 0.1 wt.-% Na₄O₇P₂ solution. 10 mL of 0.1 wt.-% Na₄O₇P₂ was added to the Hydro LV tank, then the sample slurry was introduced until an obscuration between 10-20 % was achieved. Measurements were conducted with red and blue light for 10 s each. For the analysis of the raw data, the models for non-spherical particle sizes using Mie theory was utilized, and a particle refractive index of 1.57, a density of 2.70 g/cm³, and an absorption index of 0.005 was assumed. The methods and instruments are known to the skilled person and are commonly used to determine particle size distributions of fillers and pigments.

### Specific surface area (SSA)

The specific surface area was measured via the BET method according to ISO 9277:2010 using nitrogen as adsorbing gas on a Micromeritics ASAP 2460 instrument from Micromeritics. The samples were pretreated in vacuum (10⁻⁵ bar) by heating at 150 °C for a period of 60 min prior to measurement.

### CIELAB L* of particulate materials

The CIELAB L* of the magnesium ion-containing material and other particulate materials was measured dry in accordance with EN ISO 11664-4:2010.

### Fluoride availability

A toothpaste was freshly prepared as oral care composition and aged overnight (14 h) to establish short-term equilibration of the fluoride concentration (i.e. fluoride availability) for each composition. Extraction was conducted by diluting the toothpaste with the 10-fold equivalent of demineralized water (typically 3-5 g of toothpaste diluted with 30-50 g water) in a glass beaker, followed by vigorous stirring at 800 rpm for 1 h, and filtration through a syringe filter (Chromafil Xtra, RC-20/25 0.2 µm). Fluoride availabilities were determined after volumetric dilution (Eppendorf Research Plus micropipettes) by a factor of 100 using cuvette tests (Hach Lange LCK 323, Fluoride 0.1-2.5 ppm) in a Hach-Lange DR6000 spectrophotometer. The weighted-in quantities for all dilutions were recorded and the effective (free) fluoride concentrations (i.e. the fluoride availability) in the original formulations were calculated using these values. The percentage of extractable fluoride was reported with respect to a benchmark experiments with unmodified (base formulation) toothpaste, which were conducted for each series of experiments. The result attained with the unmodified toothpaste was multiplied by 0.98 to account for the dilution of the toothpaste by the addition of the corresponding particulate material (base material). Some samples were added as filter cakes with solids contents between 10-85 wt.%, the occurring dilution was accounted for in the calculation of the fluoride availability.

### Whiteness/CIELAB L* of oral care compositions

The corresponding toothpaste was transferred into a PTFE sample holder and subsequently covered with a glass plate to attain a reproducible, flat surface. The samples were evaluated in a Datacolor ELREPHO spectrophotometer using barium sulfate as reference material. The values reported for whiteness are the L* lightness values of the CIELAB color space according to EN ISO 11664-4:2010.

### Opacity of oral care compositions

The corresponding toothpaste was diluted with 15 wt.% of demineralized water and mixed on a speed mixer (Hauschild DAC 150.1 FVZ) for 20 s at 2760 rpm. Subsequently, a 300 µm layer was spread out on a Leneta Opacity Chart (Form 3B-H) using a TQC AFA Compact automatic film applicator with 23 mm s⁻¹. The film was immediately covered with clear plastic sheets to prevent drying. The contrast value (R_{y,black}/R_{y,white}^{∗}100) was calculated based on the average of three separate measurements of R_{y} per area in a Datacolor 800V spectrophotometer using barium sulfate as reference.

### 2. Materials used

The particulate materials set out in table 1 have been used as base materials for the present invention.

**Table 1: base materials**

| **Base material** | **Name** | **Description** | **Supplier** |
|---|---|---|---|
| #M1 | PHM 1 | Precipitated hydromagnesite (PHM) | Omya International |
| #M2 | PHM 2 | PHM disagglomerated platelets | Omya International |
| #M3 | PCC 1 | Precipitated calcium carbonate | Omya International |
| #M4 | GCC | Ground calcium carbonate | Omya International |
| #M5 | PCC 2 | Precipitated calcium carbonate | Omya International |
| #M6 | PCC 3 | Precipitated calcium carbonate | Omya International |
| #M7 | TiO₂ | Titanium dioxide | Kronos |
| #M8 | MgCO₃ 1 | Magnesium carbonate basic, heavy | Sigma-Aldrich |
| #M9 | MgCO₃ 2 | Magnesium carbonate basic, light | Sigma-Aldrich |
| #M10 | MgCO₃ 4 | Wet-Milled #M11, very fine | Omya International |
| #M11 | MgCO₃ 5 | Wet-milled #M11, agglomerated | Omya International |
| #M12 | Dolomite 1 | Micronized dolomite, coarse | Omya International |
| #M13 | Dolomite 2 | Wet-milled dolomite, fine | Omya International |
| #M14 | Dolomite 3 | Wet-ground dolomite, very fine | Omya International |
| #M15 | Dolocarbonate | Dolocarbonate composite material | Omya International |
| #M16 | Mg(OH)₂ 1 | Precipitated magnesium hydroxide | Van Mannekus |
| #M17 | Mg(OH)₂ 2 | Wet-milled #M21, very fine | Omya International |

The characteristics of the base materials are set out in the following table 2.

**Table 2: characteristics of the particulate materials used as base materials**

| **Base material** | ***S*_{BET} / m²g⁻¹** | ***d*₅₀ / µm** | ***d*₉₈ / µm** |
|---|---|---|---|
| #M1 | 25 | 25 | 73 |
| #M2 | 53 | 12 | 51 |
| #M3 | 22 | 2.9 | 249 |
| #M4 | 14 | 0.3 | 3.5 |
| #M5 | 11 | 2.0 | 5.2 |
| #M6 | 11 | 2.7 | 21 |
| #M7 | 377 | 0.1 | 3.5 |
| #M8 | 9.3 | 39 | 103 |
| #M9 | 28 | 10 | 26 |
| #M10 | 35 | 0.7 | 404 |
| #M11 | 12 | 19 | 226 |
| #M12 | 3.2 | 3.3 | 11 |
| #M13 | 12 | 0.9 | 27 |
| #M14 | 17 | 0.5 | 2.7 |
| #M15 | 17 | 178 | 1250 |
| #M16 | 4.8 | 2.1 | 6.2 |
| #M17 | 35 | 0.3 | 6.1 |

For the preparation of a toothpaste base formulation, an IKA ULTRA TURRAX^{®} disperser was used. The ingredients in the toothpaste base formulation are listed in the following table 3. The formulations were prepared with 1 kg total mass. In a beaker, sorbitol, sodium fluoride, sodium saccharin, sodium benzoate, propylene glycol and glycerin and cellulose gum were vigorously mixed. Subsequently, water was added and the mixture further agitated until a homogeneous texture was attained. Then, Sorbosil AC35 was added step-wise under strong agitation and further stirred until a homogeneous texture was attained. Then, Sorbosil TC15 was added step-wise under strong agitation and further stirred until a homogeneous texture was attained to obtain the toothpaste base formulation. Two master batches of toothpaste were prepared based on different batches of raw materials. To compensate for the occurring differences (particularly in the optical properties) they will be differentiated as batch 1 (#B1) and batch 2 (#B2).

**Table 3. Recipe of the toothpaste base formulation.**

| # | **Ingredient** | **Quantity** / **mass equiv.** |
|---|---|---|
| I1 | sorbitol 70% | 23.67 |
| I2 | demineralized water | 25.85 |
| I3 | Phoskadent NaF | 0.34 |
| I4 | sodium saccharin | 0.11 |
| I5 | sodium benzoate | 0.11 |
| I6 | propylene glycol | 10.76 |
| I7 | glycerol | 10.76 |
| I8 | cellulose gum | 0.86 |
| I9 | Sorbosil AC35 silica | 21.52 |
| I10 | Sorbosil TC15 silica | 6.02 |
| I11 | sodium lauryl sulfate (15 wt.% solution) | 1.25 |
| I12 | aroma spearmint | 0.80 |

The final toothpaste was prepared in a plastic container by adding the desired quantity of the corresponding base material (0.25-2 g) to 25-30 g of the toothpaste base formulation. The formulations were manually mixed using a spatula, and subsequently homogenized using either a speed mixer (Hauschild DAC 150.1 FVZ) for 20 s at 2760 rpm or a Polytron GT 10-35 PT disperser equipped with a PT-DA 30/2EC-F250 dispersing aggregate. Subsequently, the desired quantity of surfactant (I11 according to the base formulation recipe in table 3) was added using an Eppendorf Research Plus micropipette and the formulation were mixed manually using a spatula. Finally, the desired quantity of flavour (I12 according to the base formulation recipe in table 3) was added using an Eppendorf Research Plus micropipette and the formulation was mixed manually using a spatula.

### 3. Results

The toothpastes prepared were evaluated with respect to the fluoride availability, the whiteness and opacity. The results are set out in the following table 4.

**Table 4: results**

| **Base material** | **Base formulation** | **Base material quantity / wt.%** | **Fluoride availability / %** | **Whiteness CIELAB L* / --** | **Opacity Contrast value / --.** |
|---|---|---|---|---|---|
| - | #B1 | 0 | 100 | 73.2 | 2.8 |
| - | #B2 | 0 | 100 | 79.3 | 2.7 |
| #M1 | #B1 | 1.94 | 81 | 83.5 | 5.2 |
| #M1 | #B1 | 2.94 | - | 82.1 | 7.3 |
| #M1 | #B1 | 3.80 | - | 82.3 | 9.4 |
| #M1 | #B1 | 5.82 | - | 85.1 | 12.6 |
| #M1 | #B2 | 1.87 | 82 | 84.1 | 4.7 |
| #M2 | #B2 | 1.98 | 89 | 80.8 | 4.9 |
| #M3 (ref) | #B1 | 2.03 | 46 | 85.5^{#} | 8.8^{#} |
| #M4 (ref) | #B1 | 1.95 | 39 | 86.0 | 7.5 |
| #M5 (ref) | #B1 | 1.88 | 40 | 86.1^{#} | 7.8^{#} |
| #M6 (ref) | #B1 | 1.92 | 43 | 86.0^{#} | 7.3^{#} |
| #M7 (ref) | #B1 | 0.99 | 99 | 90.4 | 20.7 |
| #M8 | #B2 | 1.96 | 86 | 83.4 | 4.0 |
| #M9 | #B2 | 1.95 | 87 | 83.2 | 3.6 |
| #M10* | #B2 | 1.83 | 100 | 84.8 | 5.8 |
| #M11* | #B2 | 1.86 | 89 | 84.1 | 4.8 |
| #M12 | #B2 | 1.92 | 94 | 84.7 | 5.5 |
| #M13* | #B2 | 1.93 | 98 | 83.0^{#} | *n*/*a^{#}* |
| #M14* | #B2 | 1.89 | 85 | 82.8^{#} | *n*/*a*^{#} |
| #M15 | #B2 | 1.98 | 81 | 84.0 | 4.2 |
| #M16 | #B2 | 1.97 | 85 | 86.3 | 7.0 |
| #M17* | #B2 | 1.91 | 89 | 83.5 | 7.5 |

| | | | | | |
|---|---|---|---|---|---|
| agglomerates were present in the toothpaste and thus no optimal data for opacity and whiteness could be achieved, may be improved by thorough mixing; * Added as filter cake | | | | | |

From the results, it can be gathered that the surface-treated materials according to the present invention provide high fluoride availability in combination with high whiteness and opacity.

## Claims

1. An oral care composition comprising a magnesium ion-containing material in an amount from 0.1 to 40 wt.-%, based on the total weight of the composition, wherein the magnesium ion-containing material is in form of particles having a volume median grain diameter (*d*₅₀) from 150 nm to 20 µm as determined according to the method specified in the description, and is selected from the group consisting of anhydrous magnesium carbonate or magnesite (MgCO₃), hydromagnesite (Mg₅(CO₃)₄(OH)₂ · 4H₂O), artinite (Mg₂(CO₃)(OH)₂ · 3H₂O), dypingite (Mg₅(CO₃)₄(OH)₂ · 5H₂O), giorgiosite (Mg₅(CO₃)₄(OH)₂ · 5H₂O), pokrovskite (Mg₂(CO₃)(OH)₂ · 0.5H₂O), barringtonite (MgCO₃ · 2H₂O), lansfordite (MgCO₃ · 5H₂O), nesquehonite (MgCO₃ · 3H₂O), brucite (Mg(OH)₂), dolomite (CaMg(CO₃)₂), dolocarbonate and mixtures thereof.

2. The oral care composition according to claim 1, wherein the magnesium ion-containing material is selected from anhydrous magnesium carbonate or magnesite (MgCO₃), dolomite (CaMg(CO₃)₂), hydromagnesite (Mg₅(CO₃)₄(OH)₂ · 4H₂O), brucite (Mg(OH)₂) and mixtures thereof.

3. The oral care composition according to claim 1 or 2, wherein the magnesium ion-containing material is in form of particles having
a) a volume median grain diameter (*d*₅₀) from 0.2 to 15 µm, even more preferably from 0.5 to 10 µm, and most preferably from 1 to 5 µm, as determined by laser diffraction, and/or
b) a volume determined top cut particle size (*d*₉₈) of equal to or less than 30 µm, preferably from 2 to 30 µm, more preferably from 5 to 20, and most preferably from 8 to 18 µm, as determined by laser diffraction.

4. The oral care composition according to any one of the preceding claims, wherein the magnesium ion-containing material has a whiteness determined as CIELAB L* of ≥ 90 %, preferably > 95 %, more preferably > 98 % and most preferably > 98.5 % and measured dry according to EN ISO 11664-4:2010.

5. The oral care composition according to any one of the preceding claims, wherein the magnesium ion-containing material is in form of particles having a BET specific surface area in the range from 2 to 200 m²/g, preferably from 10 to 100 m²/g, and most preferably from 12 to 75 m²/g, measured using nitrogen and the BET method according to ISO 9277:2010.

6. The oral care composition according to any one of the preceding claims, wherein the oral care composition further comprises a fluoride compound, preferably the fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride.

7. The oral care composition according to any one of the preceding claims, wherein the oral care composition further comprises a remineralisation and/or whitening agent, preferably selected from the group consisting of silica, hydroxylapatite, e.g. nano-hydroxylapatite, calcium carbonate, e.g. amorphous calcium carbonate, ground calcium carbonate, precipitated calcium carbonate, surface-reacted calcium carbonate and combinations thereof, calcium silicate and mixtures thereof.

8. The oral care composition according to any one of the preceding claims, wherein the oral care composition is a toothpaste, a toothgel, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash, preferably a chewable gum, a lozenge, a toothpaste, a toothpowder, or a mouthwash, and most preferably a toothpaste.

9. The oral care composition according to any one of the preceding claims, wherein the oral care composition has a pH between 6.8 and 10, preferably between 7.5 and 9 and most preferably between 8 and 9.

10. The oral care composition according to any one of the preceding claims, wherein the oral care composition comprises the magnesium ion-containing material in an amount from 0.5 to 10 wt.-%, based on the total weight of the composition.

11. Use of a magnesium ion-containing material as opacifying agent and/or whitening pigment in oral care compositions, wherein the magnesium ion-containing material is in form of particles having a volume median grain diameter (*d*₅₀) from 150 nm to 20 µm and is selected from the group consisting of anhydrous magnesium carbonate or magnesite (MgCO₃), hydromagnesite (Mg₅(CO₃)₄(OH)₂ · 4H₂O), artinite (Mg₂(CO₃)(OH)₂ · 3H₂O), dypingite (Mg₅(CO₃)₄(OH)₂ · 5H₂O), giorgiosite (Mg₅(CO₃)₄(OH)₂ · 5H₂O), pokrovskite (Mg₂(CO₃)(OH)₂ · 0.5H₂O), barringtonite (MgCO₃ · 2H₂O), lansfordite (MgCO₃ · 5H₂O), nesquehonite (MgCO₃ · 3H₂O), brucite (Mg(OH)₂), dolomite (CaMg(CO₃)₂), dolocarbonate and mixtures thereof.

12. The use according to claim 11, wherein the magnesium ion-containing material is selected from anhydrous magnesium carbonate or magnesite (MgCO₃), dolomite (CaMg(CO₃)₂), hydromagnesite (Mg₅(CO₃)₄(OH)₂ · 4H₂O), brucite (Mg(OH)₂) and mixtures thereof.

13. The use according to claim 11 or 12, wherein the magnesium ion-containing material is in form of particles having
a) a volume median grain diameter (*d*₅₀) from 0.2 to 15 µm, even more preferably from 0.5 to 10 µm, and most preferably from 1 to 5 µm, as determined by laser diffraction, and/or
b) a volume determined top cut particle size (*d*₉₈) of equal to or less than 30 µm, preferably from 2 to 30 µm, more preferably from 5 to 20, and most preferably from 8 to 18 µm, as determined by laser diffraction, and/or
c) a BET specific surface area in the range from 2 to 200 m²/g, preferably from 10 to 100 m²/g, and most preferably from 12 to 75 m²/g, measured using nitrogen and the BET method according to ISO 9277:2010.

## Patentansprüche

1. Mundpflegezusammensetzung, umfassend ein magnesiumionenhaltiges Material in einer Menge von 0,1 bis 40 Gew.-% auf Basis des Gesamtgewichts der Zusammensetzung, wobei das magnesiumionenhaltige Material die Form von Partikeln mit einem mittleren Volumen-Korndurchmesser (*d*₅₀) von 150 nm bis 20 µm aufweist, wie nach dem in der Beschreibung angegebenen Verfahren bestimmt, und ausgewählt ist aus der Gruppe bestehend aus nichtwässrigem Magnesiumcarbonat oder Magnesit (MgCO₃), Hydromagnesit (Mg₅(CO₃)₄(OH)₂ · 4H₂O), Artinit (Mg₂(CO₃)(OH)₂ · 3H₂O), Dypingit (Mg₅(CO₃)₄(OH)₂ · 5H₂O), Giorgiosit (Mg₅(CO₃)₄(OH)₂ · 5H₂O), Pokrovskit (Mg₂(CO₃)(OH)₂ · 0,5H₂O), Barringtonit (MgCO₃ · 2H₂O), Lansfordit (MgCO₃ · 5H₂O), Nesquehonit (MgCO₃ · 3H₂O), Brucit (Mg(OH)₂), Dolomit (CaMg(CO₃)₂), Dolocarbonat und Gemischen davon.

2. Mundpflegezusammensetzung nach Anspruch 1, wobei das magnesiumionenhaltige Material aus nichtwässrigem Magnesiumcarbonat oder Magnesit (MgCO₃), Dolomit (CaMg(CO₃)₂), Hydromagnesit (Mg₅(CO₃)₄(OH)₂ · 4H₂O), Brucit (Mg(OH)₂) und Gemischen davon ausgewählt ist.

3. Mundpflegezusammensetzung nach Anspruch 1 oder 2, wobei das magnesiumionenhaltige Material die Form von Partikeln mit
a) einem mittleren Volumen-Korndurchmesser (*d*₅₀) von 0,2 bis 15 µm, noch bevorzugter von 0,5 bis 10 µm und besonders bevorzugt von 1 bis 5 µm, wie durch Laserbeugung bestimmt, und/oder
b) einer volumen bestimmten Trennschnittpartikelgröße (*d*₉₈) von kleiner oder gleich 30 µm, bevorzugt von 2 bis 30 µm, bevorzugter von 5 bis 20 und besonders bevorzugt von 8 bis 18 µm, wie durch Laserbeugung bestimmt, aufweist.

4. Mundpflegezusammensetzung nach einem der vorstehenden Ansprüche, wobei das magnesiumionenhaltige Material einen als CIELAB L* von > 90 %, bevorzugt > 95 %, bevorzugter > 98 % und besonders bevorzugt > 98,5 % bestimmten und trocken nach EN ISO 11664-4:2010 gemessenen Weißgrad aufweist.

5. Mundpflegezusammensetzung nach einem der vorstehenden Ansprüche, wobei das magnesiumionenhaltige Material die Form von Partikeln mit einer BET-spezifischen Oberfläche im Bereich von 2 bis 200 m²/g, bevorzugt von 10 bis 100 m²/g und besonders bevorzugt von 12 bis 75 m²/g, gemessen unter Verwendung von Stickstoff und der BET-Methode nach ISO 9277:2010 aufweist.

6. Mundpflegezusammensetzung nach einem der vorstehenden Ansprüche, wobei die Mundpflegezusammensetzung weiter eine Fluoridverbindung umfasst, wobei die Fluoridverbindung bevorzugt aus der Gruppe bestehend aus Natriumfluorid, Zinndifluorid, Natriummonofluorphosphat, Kaliumfluorid, Kaliumzinnfluorid, Natriumfluorstannat, Zinnchlorfluorid, Aminfluorid und Gemischen davon ausgewählt ist, und bevorzugter die Fluoridverbindung Natriummonofluorphosphat und/oder Natriumfluorid ist.

7. Mundpflegezusammensetzung nach einem der vorstehenden Ansprüche, wobei die Mundpflegezusammensetzung weiter ein Remineralisations- und/oder Bleichmittel umfasst, ausgewählt bevorzugt aus der Gruppe bestehend aus Siliciumdioxid, Hydroxyapatit, z. B. Nanohydroxyapatit, Calciumcarbonat, z. B. amorphes Calciumcarbonat, gemahlenes Calciumcarbonat, ausgefälltes Calciumcarbonat, oberflächenreagiertes Calciumcarbonat und Kombinationen davon, Calciumsilicat und Gemischen davon.

8. Mundpflegezusammensetzung nach einem der vorstehenden Ansprüche, wobei die Mundpflegezusammensetzung eine Zahnpasta, ein Zahngel, ein Lack, ein Haftgel, ein Kitt, ein Harz, ein Spray, ein Schaum, ein Balsam, eine Zusammensetzung, welche auf einen Mundstreifen oder ein Wangenhaftkissen aufgebracht wird, eine Kautablette, eine Kaupastille, ein Kaugummi, eine Pastille, ein Getränk oder ein Mundwasser, bevorzugt ein Kaugummi, eine Pastille, eine Zahnpasta, ein Zahnpulver oder ein Mundwasser, und besonders bevorzugt eine Zahnpasta ist.

9. Mundpflegezusammensetzung nach einem der vorstehenden Ansprüche, wobei die Mundpflegezusammensetzung einen pH zwischen 6,8 und 10, bevorzugt zwischen 7,5 und 9 und besonders bevorzugt zwischen 8 und 9 aufweist.

10. Mundpflegezusammensetzung nach einem der vorstehenden Ansprüche, wobei die Mundpflegezusammensetzung das magnesiumionenhaltige Material in einer Menge von 0,5 bis 10 Gew.-% auf Basis des Gesamtgewichts der Zusammensetzung umfasst.

11. Verwendung eines magnesiumionenhaltigen Materials als Trübungsmittel und oder Weißpigment in Mundpflegezusammensetzungen, wobei das magnesiumionenhaltige Material die Form von Partikeln mit einem mittleren Volumen-Korndurchmesser (*d*₅₀) von 150 nm bis 20 µm aufweist, und ausgewählt ist aus der Gruppe bestehend aus nichtwässrigem Magnesiumcarbonat oder Magnesit (MgCO₃), Hydromagnesit (Mg₅(CO₃)₄(OH)₂ · 4H₂O), Artinit (Mg₂(CO₃)(OH)₂ · 3H₂O), Dypingit (Mg₅(CO₃)₄(OH)₂ · 5H₂O), Giorgiosit (Mg₅(CO₃)₄(OH)₂ · 5H₂O), Pokrovskit (Mg₂(CO₃)(OH)₂ · 0,5H₂O), Barringtonit (MgCO₃ · 2H₂O), Lansfordit (MgCO₃ · 5H₂O), Nesquehonit (MgCO₃ · 3H₂O), Brucit (Mg(OH)₂), Dolomit (CaMg(CO₃)₂), Dolocarbonat und Gemischen davon.

12. Verwendung nach Anspruch 11, wobei das magnesiumionenhaltige Material aus nichtwässrigem Magnesiumcarbonat oder Magnesit (MgCO₃), Dolomit (CaMg(CO₃)₂), Hydromagnesit (Mg₅(CO₃)₄(OH)₂ · 4H₂O), Brucit (Mg(OH)₂) und Gemischen davon ausgewählt ist.

13. Verwendung nach Anspruch 11 oder 12, wobei das magnesiumionenhaltige Material die Form von Partikeln mit
a) einem mittleren Volumen-Korndurchmesser (*d*₅₀) von 0,2 bis 15 µm, noch bevorzugter von 0,5 bis 10 µm und besonders bevorzugt von 1 bis 5 µm, wie durch Laserbeugung bestimmt, und/oder
b) einer volumen bestimmten Trennschnittpartikelgröße (*d*₉₈) von kleiner oder gleich 30 µm, bevorzugt von 2 bis 30 µm, bevorzugter von 5 bis 20 und besonders bevorzugt von 8 bis 18 µm, wie durch Laserbeugung bestimmt, und/oder
c) einer BET-spezifischen Oberfläche im Bereich von 2 bis 200 m²/g, bevorzugt von 10 bis 100 m²/g und besonders bevorzugt von 12 bis 75 m²/g, gemessen unter Verwendung von Stickstoff und der BET-Methode nach ISO 9277:2010 aufweist.

## Revendications

1. Composition d'hygiène buccale comprenant un matériau contenant des ions magnésium en une quantité de 0,1 à 40 % en poids, sur la base du poids total de la composition, dans laquelle le matériau contenant des ions magnésium est sous forme de particules présentant un diamètre de grain moyen en volume (dso) de 150 nm à 20 µm tel que déterminé selon le procédé spécifié dans la description, et est sélectionné dans le groupe consistant en du carbonate de magnésium anhydre ou de la magnésite (MgCO₃), de l'hydromagnésite (Mg₅(CO₃)₄(OH)₂ · 4H₂O), de l'artinite (Mg₂(CO₃)(OH)₂ · 3H₂O), de la dypingite (Mg₅(CO₃)₄(OH)₂ · 5H₂O), de la giorgiosite (Mg₅(CO₃)₄(OH)₂ · 5H₂O), de la pokrovskite (Mg₂(CO₃)(OH)₂ · 0.5H₂O), de la barringtonite (MgCO₃ · 2H₂O), de la lansfordite (MgCO₃ · 5H₂O), de la nesquehonite (MgCO₃ · 3H₂O), de la brucite (Mg(OH)₂), de la dolomite (CaMg(CO₃)₂), du dolocarbonate et des mélanges de ceux-ci.

2. Composition d'hygiène buccale selon la revendication 1, dans laquelle le matériau contenant des ions magnésium est sélectionné parmi du carbonate de magnésium anhydre ou de la magnésite (MgCO₃), de la dolomite (CaMg(CO₃)₂), de l'hydromagnésite (Mg₅(CO₃)₄(OH)₂ · 4H₂O), de la brucite (Mg(OH)₂) et des mélanges de ceux-ci.

3. Composition d'hygiène buccale selon la revendication 1 ou 2, dans laquelle le matériau contenant des ions magnésium est sous forme de particules présentant
a) un diamètre de grain moyen en volume (dso) de 0,2 à 15 µm, encore plus préférentiellement de 0,5 à 10 µm, et le plus préférentiellement de 1 à 5 µm, tel que déterminé par diffraction laser, et/ou
b) une taille de particules de coupe supérieure déterminée en volume (*d*₉₈) égale ou inférieure à 30 µm, de préférence de 2 à 30 µm, plus préférentiellement de 5 à 20, et le plus préférentiellement de 8 à 18 µm, telle que déterminée par diffraction laser.

4. Composition d'hygiène buccale selon l'une quelconque des revendications précédentes, dans laquelle le matériau contenant des ions magnésium présente une blancheur déterminée comme CIELAB L* > 90 %, de préférence > 95 %, plus préférentiellement > 98 % et le plus préférentiellement > 98,5 % et mesurée à sec selon EN ISO 11664-4:2010.

5. Composition d'hygiène buccale selon l'une quelconque des revendications précédentes, dans laquelle le matériau contenant des ions magnésium est sous forme de particules présentant une surface spécifique BET dans la plage de 2 à 200 m²/g, de préférence de 10 à 100 m²/g, et le plus préférentiellement de 12 à 75 m²/g, mesurée en utilisant de l'azote et la méthode BET selon ISO 9277:2010.

6. Composition d'hygiène buccale selon l'une quelconque des revendications précédentes, dans laquelle la composition d'hygiène buccale comprend en outre un composé de fluorure, de préférence le composé de fluorure est sélectionné dans le groupe consistant en du fluorure de sodium, du fluorure d'étain, du monofluorophosphate de sodium, du fluorure de potassium, du fluorure stanneux de potassium, du fluorostannate de sodium, du chlorofluorure stanneux, du fluorure d'amine, et des mélanges de ceux-ci, et plus préférentiellement le composé de fluorure est du monofluorophosphate de sodium et/ou du fluorure de sodium.

7. Composition d'hygiène buccale selon l'une quelconque des revendications précédentes, dans laquelle la composition d'hygiène buccale comprend en outre un agent de reminéralisation et/ou de blanchiment, de préférence sélectionné dans le groupe consistant en de la silice, de l'hydroxylapatite, par exemple de la nano-hydroxylapatite, du carbonate de calcium, par exemple du carbonate de calcium amorphe, du carbonate de calcium broyé, du carbonate de calcium précipité, du carbonate de calcium ayant réagi en surface et des combinaisons de ceux-ci, du silicate de calcium et des mélanges de ceux-ci.

8. Composition d'hygiène buccale selon l'une quelconque des revendications précédentes, dans laquelle la composition d'hygiène buccale est une pâte dentifrice, un gel dentifrice, une poudre dentifrice, un vernis, un gel adhésif, un ciment, une résine, un spray, une mousse, un baume, une composition réalisée sur une bande buccale ou un patch adhésif buccal, un comprimé à mâcher, une pastille à mâcher, une gomme à mâcher, une tablette, une boisson, ou un bain de bouche, de préférence une gomme à mâcher, une tablette, une pâte dentifrice, une poudre dentifrice, ou un bain de bouche, et le plus préférentiellement une pâte dentifrice.

9. Composition d'hygiène buccale selon l'une quelconque des revendications précédentes, dans laquelle la composition d'hygiène buccale présente un pH entre 6,8 et 10, de préférence entre 7,5 et 9 et le plus préférentiellement entre 8 et 9.

10. Composition d'hygiène buccale selon l'une quelconque des revendications précédentes, dans laquelle la composition d'hygiène buccale comprend le matériau contenant des ions magnésium en une quantité de 0,5 à 10 % en poids, sur la base du poids total de la composition.

11. Utilisation d'un matériau contenant des ions magnésium comme agent opacifiant et/ou pigment de blanchiment dans des compositions d'hygiène buccale, dans laquelle le matériau contenant des ions magnésium est sous forme de particules présentant un diamètre de grain moyen en volume (*d*₅₀) de 150 nm à 20 µm et est sélectionné dans le groupe consistant en du carbonate de magnésium anhydre ou de la magnésite (MgCO₃), de l'hydromagnésite (Mg₅(CO₃)₄(OH)₂ · 4H₂O), de l'artinite (Mg₂(CO₃)(OH)₂ · 3H₂O), de la dypingite (Mg₅(CO₃)₄(OH)₂ · 5H₂O), de la giorgiosite (Mg₅(CO₃)₄(OH)₂ · 5H₂O), de la pokrovskite (Mg₂(CO₃)(OH)₂ · 0.5H₂O), de la barringtonite (MgCO₃ · 2H₂O), de la lansfordite (MgCO₃ · 5H₂O), de la nesquehonite (MgCO₃ · 3H₂O), de la brucite (Mg(OH)₂), de la dolomite (CaMg(CO₃)₂), du dolocarbonate et des mélanges de ceux-ci.

12. Utilisation selon la revendication 11, dans laquelle le matériau contenant des ions magnésium est sélectionné parmi du carbonate de magnésium anhydre ou de la magnésite (MgCO₃), de la dolomite (CaMg(CO₃)₂), de l'hydromagnésite (Mg₅(CO₃)₄(OH)₂ · 4H₂O), de la brucite (Mg(OH)₂) et des mélanges de ceux-ci.

13. Utilisation selon la revendication 11 ou 12, dans laquelle le matériau contenant des ions magnésium est sous forme de particules présentant
a) un diamètre de grain moyen en volume (dso) de 0,2 à 15 µm, encore plus préférentiellement de 0,5 à 10 µm, et le plus préférentiellement de 1 à 5 µm, tel que déterminé par diffraction laser, et/ou
b) une taille de particules de coupe supérieure déterminée en volume (*d*₉₈) égale ou inférieure à 30 µm, de préférence de 2 à 30 µm, plus préférentiellement de 5 à 20, et le plus préférentiellement de 8 à 18 µm, telle que déterminée par diffraction laser, et/ou
c) une surface spécifique BET dans la plage de 2 à 200 m²/g, de préférence de 10 à 100 m²/g, et le plus préférentiellement de 12 à 75 m²/g, mesurée en utilisant de l'azote et la méthode BET selon ISO 9277:2010.
